# EUROPEAN PATENT APPLICATION

(11) **EP 3 792 627 A1**
(43) Date of publication of application: **17.03.2021**
(21) Application number: 19198717.1
(22) Date of filing: 20.09.2019
(51) Int. Cl.: G01N 33/28, G01N 21/64, G01N 33/03

(54) **A METHOD AND DEVICE FOR THE DETERMINATION OF THE ORIGIN OF AN OIL-BASED PRODUCT**

(30) Priority: 10.09.2019 PT 2019115773
(71) Applicant: Hardlevel - Energias Renováveis, S.A., 4405-516 Serzedo Vng (PT)
(72) Inventor: KARMALI, SALIM, 4405-516 SERZEDO VNG (PT); KARMALI, KARIM, 4405-516 SERZEDO VNG (PT); KARMALI, Amin, 1952-007 Lisboa, (PT)
(74) Representative: do Nascimento Gomes, Rui

(57) **Abstract**

The present invention is enclosed in the area of vegetable or animal fat oil and biodiesel control. In particular, it is directed to identifying the origin of an oil-based product which comprises a mixture of oils and/or one or more processed products obtained from oils, such oils being vegetable or animal fat oils and the one or more processed products being, for example, biofuel. The purpose of the object of the present invention thus includes to identify the presence of used cooking oils (UCOs) and used animal fats from virgin vegetable oils and virgin animal fats, thereby distinguishing their origin (used or virgin), by means of an innovative method which resorts to obtaining and analysing a spectroscopy signal of the oil-based product.

## Description

### FIELD OF THE INVENTION

The present invention is enclosed in the area of vegetable or animal fat oil and biodiesel control. In particular, it is directed to identifying the origin of an oil-based product which comprises a mixture of oils and/or one or more processed products obtained from oils, such oils being vegetable or animal fat oils and the one or more processed products being, for example, biofuel.

The purpose of the object of the present invention thus includes to identify the presence of used cooking oils (UCOs) and used animal fats from virgin vegetable oils and virgin animal fats, thereby distinguishing their origin (used or virgin).

### PRIOR ART

Large quantities of used cooking oils (UCOs) and fats are generated in several food processing industries either from plant or animal sources. Such waste residues are used as basic feedstocks for production of liquid biofuels such as biodiesel (i.e Fatty Acid Methyl Esters, FAME) and Hydro-treated Vegetable Oils or Hydro-treated esters and fatty acids (i.e HVO/HEFA) which will enhance sustainability and environmental compatibility.

Therefore, efforts have been promoted, as is the case of the European Union (EU), which has been encouraging biofuels production from waste, residues, non-food cellulosic and ligno-cellulosic materials. The Renewable Energy Directive (RED 2009/28/CE) states that waste-based biofuels can be counted twice regarding the calculation of the shares of renewable energies in transport. Therefore, it is expected that by 2020, at least 10% of transport fuels will be produced from renewable sources.

Such double counting system promotes the use of second-generation waste-based biofuels produced from feedstocks such as UCOs and Animal Fat CAT 1 and 2 which are not intended for human consumption. These raw materials represent a significant reduction in Greenhouse gas (GHG) emissions and considerable environmental advantages for the production of biofuels.

Although there are many benefits for biofuel production from such wastes, a system such as the EU system for the certification of sustainable biofuels presents some deficiencies in the production of biodiesel from waste and residues such as UCOs since there are real risks of fraud in double counting. The biofuel certified as being produced from wastes was counted double towards national targets.

However, the certification process is not fully reliable since suitable tests are lacking regarding the origin or source of the waste used. In fact, cases of fraud have been identified, by blending of virgin vegetables oils with UCOs for double counting purposes.

Therefore, it is of great interest to define waste substances for biodiesel production as well as their chemical, biological and physical characterisation. Moreover, such wastes must be characterised regarding their origin, including used or virgin, wherein a used oil may be one resulting from cooking / processing in restaurants or other food processing industries.

As far as the state of the art is concerned, there are no specific experimental methods to distinguish used cooking oils and animal fats from virgin vegetable oils and animal fats, namely for biodiesel production.

The ISCC (International Sustainability and Carbon Certification) is a traceability system which seeks to guarantee the sustainability of a product - thus trying to trace the origin of the product -, but which includes no physical evidence on the actual origin, instead consisting of a voluntary and documental system. Such comments are also applicable to other voluntary schemes that seek to ensure that biofuels comply with the EU sustainability criteria.

In fact, biofuel producers using such wastes require a generic specific assay method for these feedstocks since they have very scarce information regarding the identity and origin of waste oils obtained from different parts of the world. Moreover, it is of crucial importance to detect and quantify very low levels of UCOs and animal fats in virgin vegetable oils to avoid frauds in double counting system implemented by EU. Such a method must be use to elaborate European and International Standards to distinguish UCOs and animal fats from virgin vegetable oils and fats.

The present solution innovatively provides a solution for such problems.

### SUMMARY OF THE INVENTION

It is an object of the present invention a method for the determination of the origin of an oil-based product, the oil-based product comprising an oil, a mixture of oils and/or one or more processed products obtained from oils, wherein the oil or mixture of oils comprise vegetable or animal fat oils and the one or more processed products preferably comprise biofuel, the method comprising the steps of:
i) obtaining a spectroscopy signal of said oil-based product, wherein the spectroscopy signal is one obtained by means of a spectroscopy technique which allows to detect melanoidins, cholesterol and/or chlorophyll,
ii) comparing such spectroscopy signal with one or more predefined values of vegetable and / or animal fat oils, and
iii) determining, based on such comparison, whether such oil-based product comprises:
   - one or more oils which consist of,
      and/or
   - one or more processed products obtained from the processing of
   a used oil, such as a used cooking oil or a used animal fat, and/or a virgin vegetable or virgin animal fat oil.

Such method therefore provides for the actual determination of the origin of the oil-based product, since the analysis of a spectroscopy signal corresponding to the oil-based product - based on a technique which allows to detect melanoidins, cholesterol and/or chlorophyll - allows to identify constituents or traces/fingerprints of constituents which are only present in virgin vegetable or virgin animal fat oils, such as the presence of chlorophyll, which indicates a virgin vegetable oil, which hasn't underwent a usage procedure such as frying. It therefore indeed allows to distinguish used cooking oils and animal fats oils from virgin vegetable oils and animal fats, namely for biodiesel production. Preferably, the one or more predefined values of vegetable and / or animal fat oils comprise of one or more spectroscopy data of vegetable and / or animal fat oils. In addition, the one or more predefined values of vegetable and / or animal fat oils may also comprise a predefined set threshold.

Moreover, even where the oil-based product comprises biofuel (e.g. biodiesel), therefore being a processed product obtained from oils, the method of the present invention provides to identify through such analysis of the biofuel the same characteristic features (said traces / fingerprints) of the oils which were processed to obtain the biofuel. In other words, such features of the oils that were used to obtain the biofuel are still revealed by the analysis of the biofuel itself. As referred, the traces / fingerprints allow to identify the presence of virgin vegetable oils, virgin animal fats, used cooking oils and/or used cooking animal fats in the oils that were processed to obtain the analysed biofuel.

In addition, such determination may comprises the quantification and/or the qualitative determination of said one or more oils or one or more processed products.

It is also an object of the present invention a computational device configured to implement the method of the present invention, in any of its described embodiments.

Moreover, the present invention also includes a spectroscopy system comprising the referred computational device and a spectroscopy device which allows to detect melanoidins, cholesterol and/or chlorophyll, preferably such spectroscopy device consisting of a spectrofluorometer which performs fluorescence or phosphorescence spectroscopy.

Finally, it is also an object of the present invention a non-transitory storage media, preferably a non-transitory storage device comprising a memory, including program instructions executable to carry out the method of the present invention, in any of its described embodiments.

### DESCRIPTION OF FIGURES

The following figures make reference to samples, which are defined in the following table, the samples being of UCOs and virgin vegetable oils.

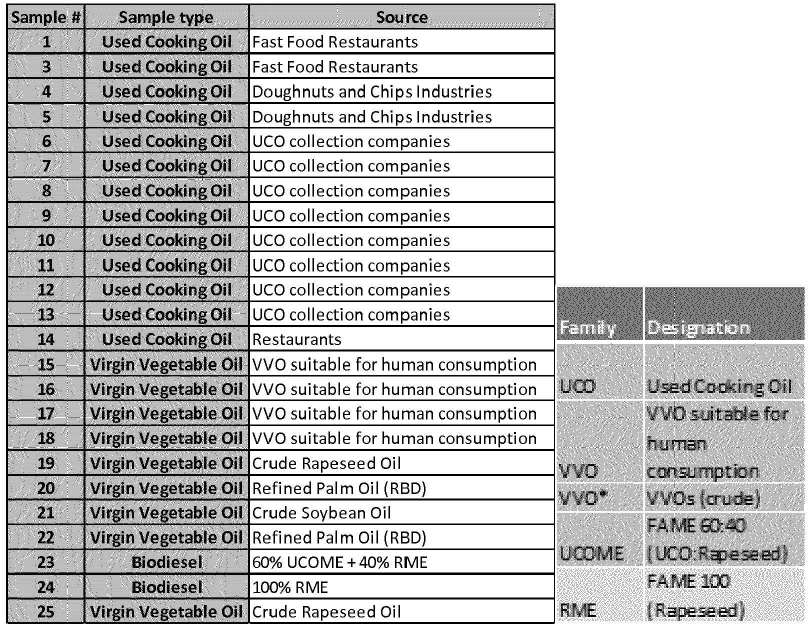

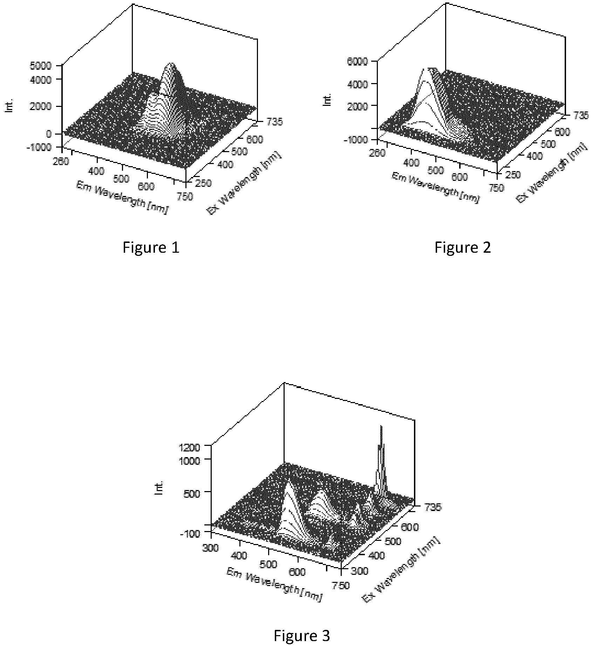

Figures 1, 2 and 3 - spectroscopy signals - spectra - of fluorescence spectroscopy of neat UCO and Virgin vegetable oils. A- S1; B-S18; C: S19.

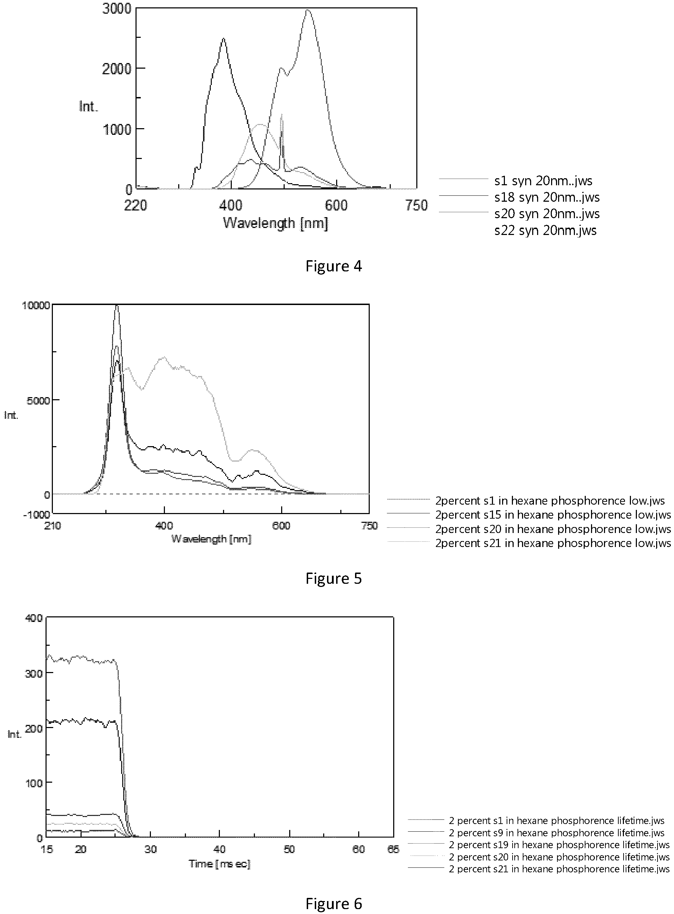

Figure 4 - Synchronous fluorescence spectroscopy of neat UCO and Virgin vegetable oils. S1, S18, S20; S22.
Figure 5 - Phosphorescence spectroscopy of UCO and Virgin vegetable oils. S1, S15, S20, S21.
Figure 6 - Phosphorescence lifetime measurement of UCO and Virgin vegetable oils. S1, S9, S19, S20, S21.

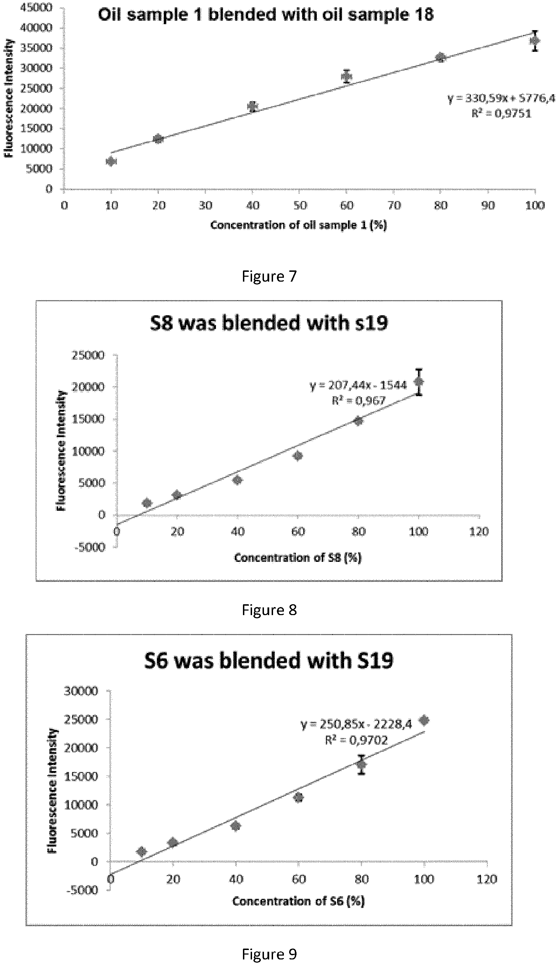

Figure 7 - Oil sample S1 was blended with S18 at different concentrations (i.e 0, 10, 20, 40, 60, 80 and 100%) by fluorescence spectroscopy in a microplate fluorescence reader.
Figure 8 - Oil sample S8 was blended with S19 at different concentrations 0, 10, 20, 40, 60, 80 and 100% by synchronous fluorescence spectroscopy.
Figure 9 - Oil sample S6 was blended with S19 at different concentrations 0, 10, 20, 40, 60, 80 and 100% by synchronous fluorescence spectroscopy.

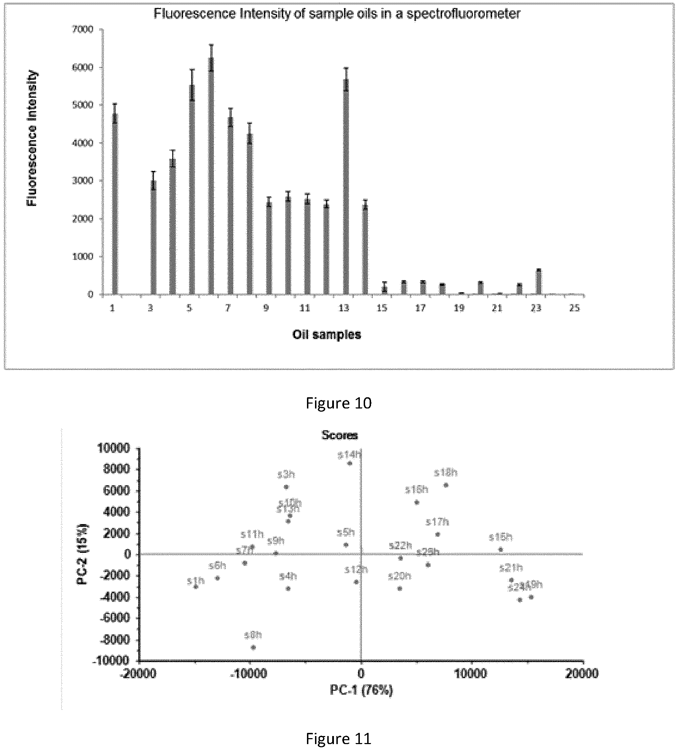

Figure 10 - Synchronous fluorescence intensity of all neat oils in a spectrofluorometer
Figure 11 - PCA of all oils (0.1%) in hexane based on 3-D spectra by using Unscrambler version 10.4 (64 bits).

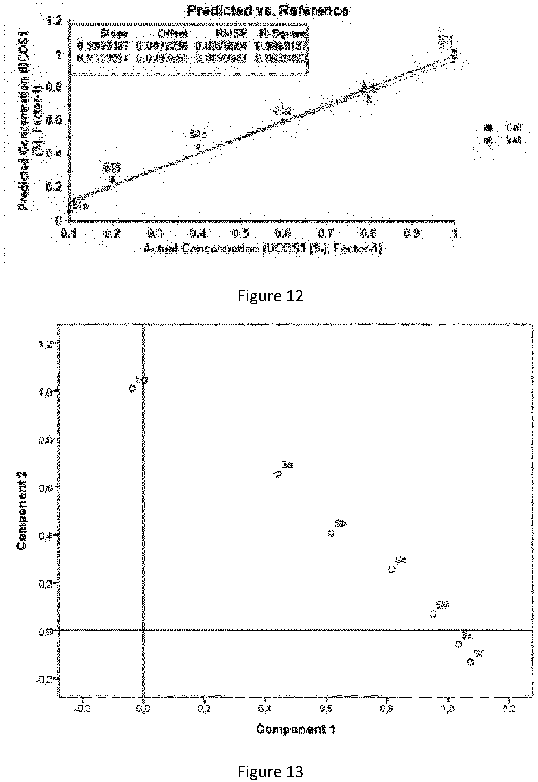

Figure 12 - Predicted versus actual concentration of UCOs S1 in virgin vegetable oil S19.
Figure 13 - PCA of S6 blended with s19 at different concentrations which were analysed by synchronous fluorescence spectroscopy at 7.5 nm delta wavelength. ; Sa-0.1% S6 blended with S19; Sb- 0.2% S6 blended with S19; Sc- 0.4% S6 blended with S19; Sd- 0.6% S6 blended with S19; Se - 0.8% S6 blended with S19; Sf - 1% S6; Sg - 1% S19. Component 1 (87.098 %) and Component 2 (9.97%).

### DETAILED DESCRIPTION

The more general and advantageous configurations of the present invention are described in the Summary of the invention. Such configurations are detailed below in accordance with other advantageous and/or preferred embodiments of implementation of the present invention.

In an inventive aspect of the method of the present invention, the predefined spectroscopy data of step ii) comprises spectroscopy data corresponding to compounds or mixtures which contain melanoidins, cholesterol and/or chlorophyll. It therefore allows to focus the comparison only on the identification of compounds or mixtures which contain melanoidins, cholesterol and/or chlorophyll, therefore simplifying and speeding the identification of the presence virgin vegetable oils, virgin animal fats, used cooking oils and/or used cooking animal fats.

In another relevant inventive aspect of the method of the present invention, the comparison of step ii) between the obtained spectroscopy signal and the predefined values, such values preferably consisting of spectroscopy data, comprises the comparison of at least one intensity value of the obtained spectroscopy signal with a predefined threshold value of predefined spectroscopy data and, if the value of the obtained spectroscopy signal is above the predefined threshold value, determining that the:
- one or more oils consist of,
   and/or
- one or more processed products are obtained from the processing of
a used oil, such as a used cooking oil or a used animal fat, and/or virgin vegetable or virgin animal fat oil.

Such enhancement provides for a specific and very reliable indicator of the presence of a used cooking oil or a used animal fat or a virgin vegetable or virgin animal fat oil, relying only in the analysis of peaks of the obtained spectroscopy signal, by comparing them with a predefined threshold value of predefined spectroscopy data. The occurrence of such peaks, as is experimentally demonstrated in a subsequent section, is a fingerprint of certain compounds or mixtures, and identifies the presence of used cooking oil or a used animal fat or a virgin vegetable or virgin animal fat oils. For instance, a high intensity in a fluorescence technique may indicate the presence of used oils. Further data is provided in the experimental data section. An analysis of peaks in specific previously identified wavelengths provides for an indication on the presence of chlorophyll.

In addition, the method may further comprise that the predefined spectroscopy data of the comparison of step ii) further comprises at least one predefined spectroscopy signal of vegetable and / or animal fat oils. In such case, actual predefined spectroscopy signals, namely spectroscopy signals which were previously obtained and analysed in order to identify the corresponding constituents and in particular their origin, are used to determine the origin of the oil-based product under analysis, in a more reliable way.

In a preferred embodiment of the method of the present invention, the spectroscopy technique consists of a fluorescence or phosphorescence spectroscopy, which provides an enhanced comparison between the spectroscopy signal and the predefined spectroscopy data, in particular where an intensity value of the obtained spectroscopy signal is compared with a predefined threshold value of predefined spectroscopy data.

In a particular embodiment, such fluorescence or phosphorescence spectroscopy is synchronous, and the spectroscopy signal has been obtained by means of a spectrofluorometer.

Such provides for a difference in intensity for the various UCO Vegetable Oils, as well as an identification of the characteristic markers (the previously mentioned fingerprints / traces) of the presence of each of the oils (shown in the 2D and 3D phosphorescence spectra, where the characteristic markers intensity are obtained as a function of wavelength).

Moreover, a fluorometric spectrum technique allows the identification of specific peak location regions characteristic for each sample, namely for location and statistical identification.

In yet another embodiment of the method of the present invention, the synchronous fluorescence or phosphorescence spectroscopy is performed using different delta wavelengths.

In particular, where the spectroscopy signal has been obtained by means of a spectrofluorometer, the spectroscopy technique consists of a phosphorescence spectroscopy which provides lifetime measurements, thereby the spectroscopy signal comprising such measurements.

Also as regards the embodiment in which the spectroscopy signal has been obtained by means of a spectrofluorometer, the spectroscopy technique preferably provides 3-D spectra measurements, thereby the spectroscopy signal comprising such measurements.

Optionally, where the spectroscopy technique consists of a fluorescence spectroscopy, the spectroscopy signal has been obtained by means of a microtiter plate reader. In another optional embodiment, the fluorescence spectroscopy is time-resolved.

In yet another optional embodiment of the method of the present invention, and as regards the comparison of step ii), the comparison of step ii) is performed by means of a statistical procedure between the obtained spectroscopy signal and the predefined spectroscopy data, in particular the statistical procedure comprising one or more of the following: principal component analysis, partial least square regression, Soft Independent Modelling of Class Analogy and/or linear discriminant analysis.

### EMBODIMENTS AND EXPERIMENTAL DATA

Subsequently, several embodiments of the method of the present invention and corresponding experimental results are presented.

In particular, such embodiments comprise the analysis of several variants of fluorescence spectroscopy to distinguish UCOs and animal fats from virgin vegetable oils and fats.

These variants were previously referred, and are synchronous fluorescence, phosphorescence, phosphorescence time resolved, 3-D spectra, time resolved fluorescence spectroscopy either in microtiter plate reader or in a spectrofluorometer.

The samples were analysed either by using neat oils or oils diluted in hexane. A database of several oils was built by using statistical analysis and therefore IBM Statistics SPSS version 23 and Unscrambler version 10.4 were used for PCA, PLS, SIMCA and LDA. These results strongly suggest that it is possible to detect oil blendings with a detection limit of 0.18%.

The chemical and physical changes that occur in vegetable oils and fats during deep-frying process can be monitored and quantified since there are increased levels of free fatty acids, polar compounds (ISO 8420: 2002), colour viscosity, polymerized triacylglycerols (ISO 16931: 2009), volatile compounds, peroxides and foam whereas there is a marked decrease in the degree of unsaturation of fatty acids. However, these methods, are non-specific, tedious, time consuming and highly unreliable.

The list of oil samples used in the herein described experimental setups us presented Table 1.

The oils samples were read either in microtiter plate fluorescence reader or spectrofluorometer by using either neat oils or oils diluted in suitable solvents (i.e hexane, isopropyl alcohol, heptane, chloroform and pentane) A Jasco spectrofluorometer (Japan) with a Model FP-8300, and 1 cm quartz cuvettes were used. The fluorescent emission spectra of the samples were obtained by using excitation bandwidth of 5 nm, emission bandwidth of 5 nm, response of 50 msec, medium sensitivity, excitation wavelength of 485 nm, measurement range of 250 - 750 nm, data interval of 0.2 nm and scan speed of 500 nm/min. The spectra data were generated by using spectra manager. Fluorescence measurements of each oil sample was carried out by using a Fluostar Optima (BMG) microtiter plate fluorimeter at excitation wavelength of 485 and emission wavelength of 528 nm and gain of 1200. Time resolved fluorescence measurements were carried out by at same excitation and emission wavelengths at integration time of 15 to 1500 ms and gain of 1200. fluorescence Phosphorescence synchronous spectroscopy was carried out at excitation bandwidth of 20 nm, emission bandwidth of 20 nm, low sensitivity, chopping period of 100 msec, delay time of 10 msec, integration time of 65 msec, response time of 0.2 sec, measurement range of 210 - 750 nm, data interval of 2 nm and appropriate delta wavelengths.

Phosphorescence lifetime measurements were carried out by phosphorescence intensity, excitation bandwidth of 5 nm, emission bandwidth of 5 nm, medium sensitivity, chopping period of 100 msec, and measurement range of 0 - 75 msec, data interval of 0.1 msec, delay time of 15 msec, excitation wavelength of 485.0 nm and emission wavelength of 540.0 nm.

3-D spectra measurements were carried out in a spectrofluorometer in the range of 260 - 750 nm with a data interval of 0.5 nm, excitation bandwidth of 5 nm, emission bandwidth of 5 nm, Response time of 10 msec, high sensitivity, excitation wavelength of 250.0 nm and scan speed of 10000 nm/min.

Statistical analysis was used to build a database for several oils by using spss 23 and Unscrambler version 10.4. Principal component analysis (PCA), partial least square regression (PLS), Soft Independent Modeling of Class Analogy (SIMCA) and linear discriminant analysis (LDA).

The increase in fluorescence of UCOs and animal fats compared to virgin vegetable and animal fats is due to the presence of melanoidins, several types of polar compounds such as acrylamide and polymeric compounds such as polymerized triacylglycerols (data not shown).

### Example1

Either neat oils or oils diluted 1:100 in hexane were used to measure fluorescence intensity in the microtiter plate fluorescence reader as well as in spectrofluorometer by emission fluorescence intensity and 3-D spectra measurements as described in section of detailed description. Briefly, 0.1ml of either neat oils or diluted in hexane were placed in 96 well microtiter plates in triplicates and they were read in microtiter plate fluorescence reader at room temperature. Either empty wells or wells containing hexane were used as the controls. As far as spectrofluorometer is concerned, 3 ml of either neat oils or oils diluted in hexane were used in quartz cuvettes which were placed in the sample compartment and measurements were carried out at room temperature. A blank cuvette was used as a control. All measurements were carried out in triplicates. Statistical analysis of the data was carried out by IBM Statistics SPSS version 23 as well as The Unscrambler version 10.4 (64 bits). PCA, PLS, SIMCA and LDA were used to analyse spectral data.

### Example 2

Either neat oils or oils diluted 1:100 in hexane were used to measure time resolved fluorescence intensity in the microtiter plate fluorescence reader. Synchronous fluorescence intensity and synchronous phosphorescence intensity were measured in spectrofluorometer by using different delta wavelengths as well as phosphorescence lifetime measurements as described in section of detailed description. Briefly, 0.1ml of either neat oils or diluted in hexane were placed in 96 well microtiter plates in triplicates and they were read in microtiter plate fluorescence reader at room temperature. Either empty wells or wells containing hexane were used as the controls. As far as spectrofluorometer is concerned, 3 ml of either neat oils or oils diluted in hexane were used in quartz cuvettes which were placed in the sample compartment and measurements were carried out at room temperature. A blank cuvette was used as a control. All measurements were carried out in triplicates. Statistical analysis of the data was carried out by IBM Statistics SPSS version 23 as well as The Unscrambler version 10.4 (64 bits). PCA, PLS, SIMCA and LDA were used to analyse spectral data.

### Example 3

Blending of UCOs (S1) with virgin vegetable oils (S18) by using either neat oils or diluted in hexane were used to measure time resolved fluorescence intensity in the microtiter plate fluorescence reader. Synchronous fluorescence intensity and synchronous phosphorescence intensity were measured in spectrofluorometer by using different delta wavelengths as well as phosphorescence lifetime measurements as described in section of detailed description. Briefly, 0.1ml of either neat oils or diluted in hexane were placed in 96 well microtiter plates in triplicates and they were read in microtiter plate fluorescence reader at room temperature. Either empty wells or wells containing hexane were used as the controls. As far as spectrofluorometer is concerned, 3 ml of either neat oils or oils diluted in hexane were used in quartz cuvettes which were placed in the sample compartment and measurements were carried out at room temperature. A blank cuvette was used as a control. All measurements were carried out in triplicates. Statistical analysis of the data was carried out by IBM Statistics SPSS version 23 as well as The Unscrambler version 10.4 (64 bits). PCA, PLS, SIMCA and LDA were used to analyse spectral data.

### Example 4

Blending of UCOs (S1) with virgin vegetable oils (S18) by using either neat oils or diluted in hexane were used to measure fluorescence intensity in the microtiter plate fluorescence reader and emission fluorescence intensity in spectrofluorometer as well as 3-D- spectra as described in section of detailed description. Briefly, 0.1ml of either neat oils or diluted in hexane were placed in 96 well microtiter plates in triplicates and they were read in microtiter plate fluorescence reader at room temperature. Either empty wells or wells containing hexane were used as the controls. As far as spectrofluorometer is concerned, 3 ml of either neat oils or oils diluted in hexane were used in quartz cuvettes which were placed in the sample compartment and measurements were carried out at room temperature. A blank cuvette was used as a control. All measurements were carried out in triplicates. Statistical analysis of the data was carried out by IBM Statistics SPSS version 23 as well as The Unscrambler version 10.4 (64 bits). PCA, PLS, SIMCA and LDA were used to analyse spectral data.

### Example 5

Blending of UCOs (S5) with virgin vegetable oils (S19) by using either neat oils or diluted in hexane were used to measure fluorescence intensity in the microtiter plate fluorescence reader and emission fluorescence intensity in spectrofluorometer as well as 3-D- spectra as described in section of detailed description. Briefly, 0.1ml of either neat oils or diluted in hexane were placed in 96 well microtiter plates in triplicates and they were read in microtiter plate fluorescence reader at room temperature. Either empty wells or wells containing hexane were used as the controls. As far as spectrofluorometer is concerned, 3 ml of either neat oils or oils diluted in hexane were used in quartz cuvettes which were placed in the sample compartment and measurements were carried out at room temperature. A blank cuvette was used as a control. All measurements were carried out in triplicates. Statistical analysis of the data was carried out by IBM Statistics SPSS version 23 as well as The Unscrambler version 10.4 (64 bits). PCA, PLS, SIMCA and LDA were used to analyse spectral data.

### Example 6

Blending of UCOs (S5) with virgin vegetable oils (S19) by using either neat oils or diluted in hexane were used to measure time resolved fluorescence intensity in the microtiter plate fluorescence reader with an integration time of 15 ms and 50 ms and gain of 1200. Synchronous fluorescence intensity and synchronous phosphorescence intensity were measured in spectrofluorometer by using different delta wavelengths as well as phosphorescence lifetime measurements as described in section of detailed description. Briefly, 0.1ml of either neat oils or diluted in hexane were placed in 96 well microtiter plates in triplicates and they were read in microtiter plate fluorescence reader at room temperature. Either empty wells or wells containing hexane were used as the controls. As far as spectrofluorometer is concerned, 3 ml of either neat oils or oils diluted in hexane were used in quartz cuvettes which were placed in the sample compartment and measurements were carried out at room temperature. A blank cuvette was used as a control. All measurements were carried out in triplicates. Statistical analysis of the data was carried out by IBM Statistics SPSS version 23 as well as The Unscrambler version 10.4 (64 bits). PCA, PLS, SIMCA and LDA were used to analyse spectral data.

As will be clear to one skilled in the art, the present invention should not be limited to the embodiments described herein, and a number of changes are possible which remain within the scope of the present invention.

Of course, the preferred embodiments shown above are combinable, in the different possible forms, being herein avoided the repetition all such combinations.

## Claims

1. A method for the determination of the origin of an oil-based product, the oil-based product comprising an oil, a mixture of oils and/or one or more processed products obtained from oils, wherein the oil or mixture of oils comprise vegetable or animal fat oils and the one or more processed products preferably comprise biofuel, the method being **characterised in that** it comprises the steps of:
i) obtaining a spectroscopy signal of said oil-based product, wherein the spectroscopy signal is one obtained by means of a spectroscopy technique which allows to detect melanoidins, cholesterol and/or chlorophyll,
ii) comparing such spectroscopy signal with one or more predefined values of vegetable and / or animal fat oils, and
iii) determining, based on such comparison, whether such oil-based product comprises:
- one or more oils which consist of,
and/or
- one or more processed products obtained from the processing of
a used oil, such as a used cooking oil or a used animal fat, and/or a virgin vegetable or virgin animal fat oil.

2. A method according to the previous claim wherein said determination comprises the quantification and/or the qualitative determination of said one or more oils or one or more processed products.

3. A method according to any of the preceding claims wherein the one or more predefined values of vegetable and / or animal fat oils consist of one or more spectroscopy data of vegetable and / or animal fat oils.

4. A method according to the previous claim wherein the predefined spectroscopy data of step ii) comprises spectroscopy data corresponding to compounds or mixtures which contain melanoidins, cholesterol and/or chlorophyll.

5. A method according to any of the claims 3-4 wherein the comparison of step ii) between the obtained spectroscopy signal and the predefined spectroscopy data comprises the comparison of at least one intensity value of the obtained spectroscopy signal with a predefined threshold value of predefined spectroscopy data and, if the value of the obtained spectroscopy signal is above the predefined threshold value, determining that the:
- one or more oils consist of,
and/or
- one or more processed products are obtained from the processing of
a used oil, such as a used cooking oil or a used animal fat, and/or a virgin vegetable or virgin animal fat oil.

6. A method according to any of the claims 3-5 wherein the predefined spectroscopy data of the comparison of step ii) further comprises at least one predefined spectroscopy signal of vegetable and / or animal fat oils.

7. A method according to any of the claims 3-6 wherein the spectroscopy technique consists of a fluorescence or phosphorescence spectroscopy.

8. A method according to the previous claim wherein such fluorescence or phosphorescence spectroscopy is synchronous, and the spectroscopy signal has been obtained by means of a spectrofluorometer wherein, preferably, such synchronous fluorescence or phosphorescence spectroscopy is performed using different delta wavelengths.

9. A method according to claim 8 wherein the spectroscopy technique consists of a phosphorescence spectroscopy which provides lifetime measurements, thereby the spectroscopy signal comprising such measurements.

10. A method according to any of the claims 8-9 wherein the spectroscopy technique provides 3-D spectra measurements, thereby the spectroscopy signal comprising such measurements.

11. A method according to claim 7 wherein the spectroscopy technique consists of a fluorescence spectroscopy and the spectroscopy signal has been obtained by means of a microtiter plate reader and, optionally, the fluorescence spectroscopy is time-resolved.

12. A method according to any of the claims 3-11 wherein the comparison of step ii) is performed by means of a statistical procedure between the obtained spectroscopy signal and the predefined spectroscopy data, in particular the statistical procedure comprising one or more of the following: principal component analysis, partial least square regression, Soft Independent Modelling of Class Analogy and/or linear discriminant analysis.

13. A computational device configured to implement the method of any of the preceding claims.

14. A spectroscopy system comprising the computational device of the previous claim, in particular a computational device configured to implement the method of any of the claims 3-12, and a spectroscopy device which allows to detect melanoidins, cholesterol and/or chlorophyll, preferably such spectroscopy device consisting of a spectrofluorometer which performs fluorescence or phosphorescence spectroscopy.

15. Non-transitory storage media, preferably a non-transitory storage device comprising a memory, including program instructions executable to carry out the method of any of the claims 1-12.
